# EUROPEAN PATENT SPECIFICATION

(11) **EP 4 394 353 B1**
(45) Date of publication and mention of the grant of the patent: **11.11.2026**
(21) Application number: 22875935.3
(22) Date of filing: 20.09.2022
(51) Int. Cl.: G01N 21/17, C10B 57/04, G01N 21/47, G01N 1/32, G01N 21/55, G01N 21/84, C10B 45/00, G01N 21/27, G01N 1/28, G01N 1/36

(54) **COAL ANALYZER, COAL ANALYSIS METHOD, MIXED COAL PREPARATION METHOD, AND COKE PRODUCTION METHOD**
KOHLEANALYSEGERÄT, KOHLEANALYSEVERFAHREN, MISCHKOHLEHERSTELLUNGSVERFAHREN UND KOKSHERSTELLUNGSVERFAHREN
ANALYSEUR DE CHARBON, PROCÉDÉ D'ANALYSE DE CHARBON, PROCÉDÉ DE PRÉPARATION DE CHARBON MÉLANGÉ ET PROCÉDÉ DE PRODUCTION DE COKE

(30) Priority: 30.09.2021 JP 2021161012
(43) Date of publication of application: 03.07.2024
(73) Proprietor: JFE Steel Corporation, Tokyo 100-0011 (JP)
(72) Inventor: TSUBOI Toshiki, Tokyo 100-0011 (JP); KAMEZAKI Shunichi, Tokyo 100-0011 (JP)
(74) Representative: Hoffmann Eitle
(86) International application number: PCT/JP2022/034999
(87) International publication number: WO 2023/054065

(56) References cited:
- CN-A- 102 297 850
- CN-A- 111 160 064
- CN-A- 111 879 732
- JP-A- 2005 338 011
- JP-A- S5 822 940
- JP-A- S50 112 096
- JP-A- S58 153 144
- US-A- 5 841 882

## Description

### TECHNICAL FIELD

The present invention relates to a coal analyzer, a coal analysis method, a mixed coal preparation method, and a coke production method.

### BACKGROUND ART

Coke is produced from coal with various brands blended according to its product indexes.

Coal used for coke production is analyzed in advance for the structural components (Patent Literature 1).

US 5,841,882 A discloses a process for the maceral analysis of a sample of crushed coal coated with resin. The process includes the steps of scanning the surface of the sample with a microscope and measuring the reflectance of the vitrinite. The process further includes the steps of forming a digitized image having gray levels for each field of the microscope and eliminating those fields having a high resin content. In those fields which are not eliminated, the distribution of gray levels and gradients are determined, a binary image is formed by thresholding the gray gradients to determine zones of vitrinite, the distribution of the gray levels of the obtained zones of vitrinite are then determined and the reflectance of the vitrinite is calculated.

### CITATION LIST

### PATENT LITERATURE

Patent Literature 1: JP S58-35442 A

### SUMMARY OF INVENTION

### TECHNICAL PROBLEMS

One of important product indexes of coke is cold coke strength.

There are three types of microstructural component groups (maceral groups) of coal: vitrinite, exinite, and inertinite. Among them, for example, inertinite is chemically inert and affects the cold coke strength.

For this reason, the ratio of inertinite (total inert amount) in coal used for coke production is measured.

Conventionally, the total inert amount of coal is measured using a microscope in accordance with JIS M 8816-1992 (Solid mineral fuels-Methods of microscopical measurement for the macerals and reflectance).

In this case, it is necessary to individually identify a microstructural component (maceral) belonging to inertinite such as semi-fusinite, thereby making the measurement very complicated. In addition, since the identification is performed based on a criterion such as a pattern, the identification result is largely dependent on a person, and such identification is also time-consuming.

The same applies to the measurement of the ratios of vitrinite and exinite, which are the other microstructural component groups.

The present invention has been made in view of the above points, and an object thereof is to easily obtain a ratio of at least one of the microstructural component groups of coal.

### SOLUTION TO PROBLEMS

As a result of intensive studies, the present inventors have found that the above object is achieved by adopting the following constitution, as defined in the appended claims.

### ADVANTAGEOUS EFFECTS OF INVENTION

According to the present invention, the ratio of at least one of the microstructural component groups of coal can be easily obtained.

### BRIEF DESCRIPTION OF DRAWINGS

[FIG. 1] FIG. 1 is a block diagram illustrating a configuration of a coal analyzer.
[FIG. 2] FIG. 2 is a flowchart illustrating a flow of processing executed by respective units included in the coal analyzer.
[FIG. 3] FIG. 3 is a view illustrating a surface image of a coal sample.
[FIG. 4] FIG. 4 is an aggregate reflectance histogram of all coal particles.
[FIG. 5] FIG. 5 is a reflectance histogram of one coal particle.
[FIG. 6] FIG. 6 is a relationship diagram between Ro (manual) and Ro (automatic).
[FIG. 7] FIG. 7 is a relationship diagram between a total inert amount (manual) and a total inert amount (automatic).
[FIG. 8] FIG. 8 is a reflectance histogram of one coal particle, which histogram illustrates a semi-fusinite reflectance.
[FIG. 9] FIG. 9 is a relationship diagram between Ro (automatic) and a semi-fusinite ratio (manual).
[FIG. 10] FIG. 10 is a relationship diagram between the total inert amount (manual) and a corrected total inert amount (automatic).

### DESCRIPTION OF EMBODIMENTS

### [Coal (microstructural component and microstructural component group)]

Coal is composed of microstructural components having different gloss and shape under a microscope.

A microstructural component (maceral) and a microstructural component group (maceral group) of coal will be described.

The microstructural component (maceral) of coal is a fine organic component of coal that is classified into 12 types with a microscope.

Specifically, the 12 types of microstructural components are telinite, collinite, degradinite, sporinite, cutinite, alginite, resinite, semi-fusinite, fusinite, micrinite, sclerotinite, and macrinite.

The microstructural component group (maceral group) of coal is a component group of microstructural components of coal having similar properties under a microscope, and is classified into three types.

Specifically, the three types of microstructural component groups are vitrinite, exinite, and inertinite (also referred to as "inert").

Microstructural components belonging to vitrinite are three types: telinite, collinite, and degradinite.

Microstructural components belonging to exinite are four types: sporinite, cutinite, alginite, and resinite.

Microstructural components belonging to inertinite are five types: semi-fusinite, fusinite, micrinite, sclerotinite, and macrinite.

In general, a total inert amount representing the ratio of inertinite among the three types of microstructural components (vitrinite, exinite, and inertinite) is used for the design of cold coke strength.

For this reason, the total inert amount of coal is obtained in the present embodiment.

### [Coal analyzer (coal analysis method)]

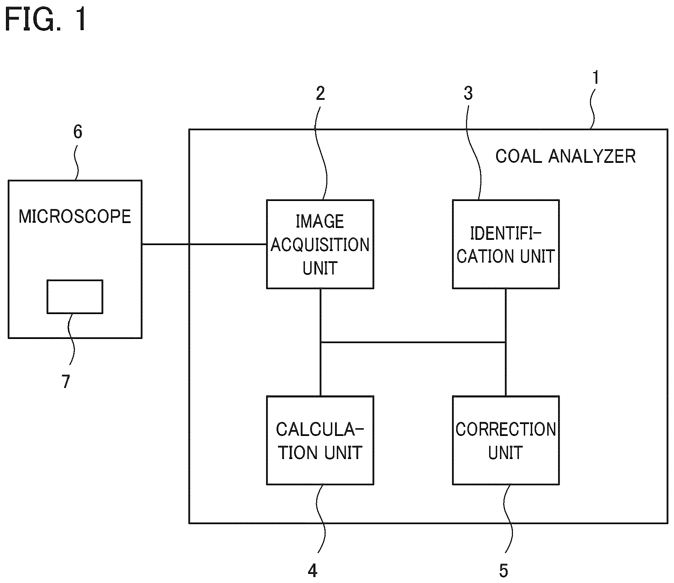

Hereinafter, a coal analyzer of the present embodiment will be described with reference to FIGS. 1 to 10. The following description doubles as a description of the coal analysis method of the present embodiment.

### <Configuration>

FIG. 1 is a block diagram illustrating a configuration of a coal analyzer 1.

The coal analyzer 1 includes an image acquisition unit 2, an identification unit 3, a calculation unit 4, and a correction unit 5.

The image acquisition unit 2, the identification unit 3, the calculation unit 4, and the correction unit 5 are composed of, for example, a central processing unit (CPU), a main storage device such as a dynamic random access memory (DRAM) connected to the CPU, a mass storage device such as a solid state drive (SSD) or a hard disk drive (HDD) connected to the CPU, and a program for operating the CPU.

Processing executed by these units will be described later.

The coal analyzer 1 is connected to a microscope 6. A coal sample 7 is placed on a stage (not shown) of the microscope 6.

The coal sample 7 is prepared in accordance with JIS M 8816-1992.

More specifically, the coal sample 7 is obtained by pulverizing the collected coal, embedding the obtained coal particles in a resin, curing the resin, and then polishing the surface of the cured object.

The microscope 6 captures a surface image of the coal sample 7.

It is preferable that the microscope 6 has a function of designating a macro imaging range in advance, capturing a plurality of micro images while automatically moving the stage, and finally combining the captured micro images into a macro image.

In the present embodiment, for example, the size of the micro image is about 800 µm in length and 530 µm in width, and an oil immersion objective lens (magnification: 20 times) is used as the objective lens.

However, as long as the surface image of the coal sample 7 can be captured, the microscope 6 may not have the above function, and another objective lens may be used.

### <Processing of respective units>

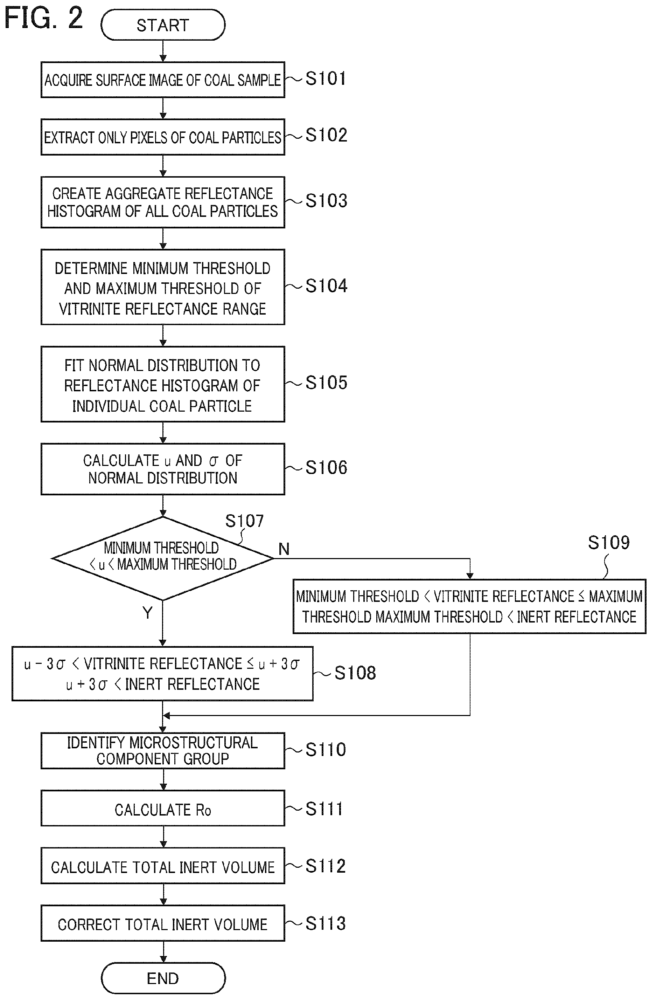

FIG. 2 is a flowchart illustrating a flow of processing executed by respective units included in the coal analyzer 1.

### <<Image acquisition unit>>

First, the image acquisition unit 2 of the coal analyzer 1 causes the microscope 6 to capture a surface image of the coal sample 7, and acquires the captured surface image (step S101).

Only one surface image of the coal sample 7 may be captured to calculate the total inert amount, but only one surface image is merely part of the surface of the coal sample 7, and is thus less representative. To ensure the representativeness, it is preferable to image a plurality of portions of the surface of the coal sample 7, and it is more preferable to image all the portions of the surface (entire surface) of the coal sample 7.

In the present embodiment, the entire surface of the coal sample 7 is imaged to obtain about 2000 individual images, and the obtained individual images are combined to obtain one huge surface image.

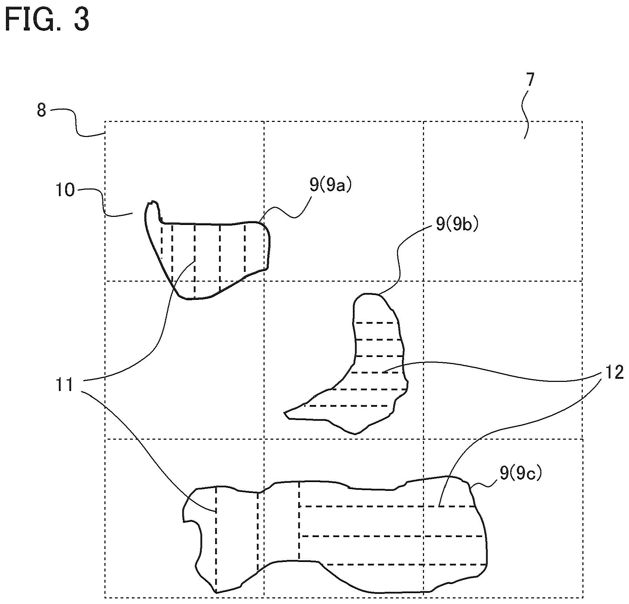

FIG. 3 is a view illustrating a surface image 8 of the coal sample 7. In FIG. 3, for simplification, nine individual images are combined to form one surface image 8.

In the surface image 8 of FIG. 3, three coal particles 9 (coal particle 9a, coal particle 9b, and coal particle 9c) embedded in the resin 10 are partly exposed.

In FIG. 3, vitrinite 11 is indicated by a vertical broken line, and inert 12 is indicated by a horizontal broken line.

The coal particle 9a in the upper part of the surface image 8 is formed of the vitrinite 11.

The coal particle 9b in the middle part of the surface image 8 is formed of the inert 12.

The coal particle 9c in the lower part of the surface image 8 is a mixture of the vitrinite 11 and the inert 12.

### <<Identification unit>>

Next, the identification unit 3 of the coal analyzer 1 extracts only pixels corresponding to the coal particles 9 from the surface image 8 (step S102).

In the surface image 8, the resin 10 takes on a blackish color. Thus, for example, the surface image 8 is subjected to differential processing, a boundary between the coal particle 9 and the resin 10, which has a large differential value, is detected, and only each pixel inside the boundary (that is, the coal particle 9) is acquired.

Each pixel constituting the surface image 8 includes luminance information.

In the present embodiment, a calibration curve between luminance and reflectance is created in advance using a standard test piece with known reflectance. The identification unit 3 of the coal analyzer 1 converts the luminance of each pixel into reflectance based on the created calibration curve. In this manner, the reflectance of each pixel of the coal particle 9 is obtained.

Next, the identification unit 3 of the coal analyzer 1 collects and aggregates the reflectances of the respective pixels to create an aggregate reflectance histogram of all the coal particles (step S103).

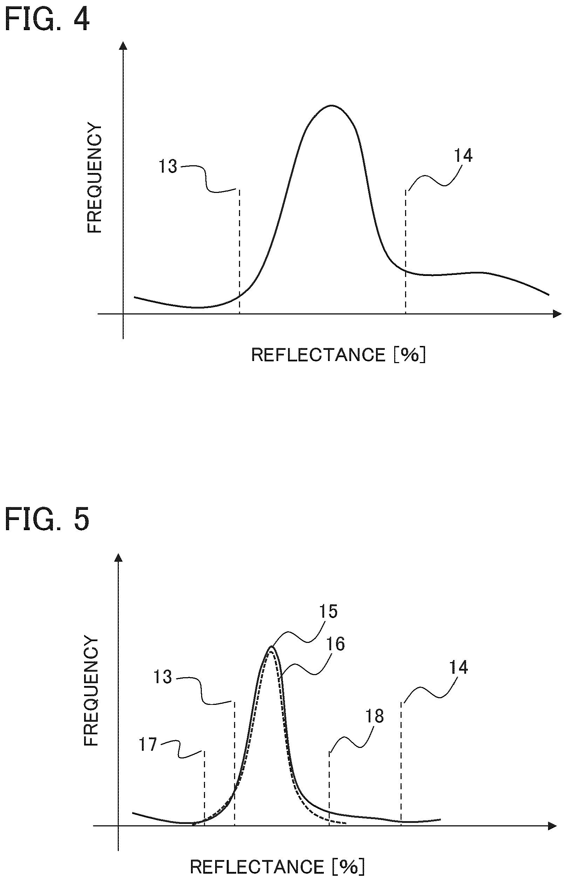

FIG. 4 is an aggregate reflectance histogram of all the coal particles. The horizontal axis represents reflectance (unit: %), and the vertical axis represents frequency (number of cases) (the same applies hereinafter).

The reflectances of vitrinite, exinite, and inert have a magnitude relationship: inert > vitrinite > exinite.

In general, the reflectance distribution of vitrinite is a normal distribution.

The histogram of FIG. 4 is formed by combining a mountain of vitrinite having a normal distribution shape with a mountain of inert having a long skirt in a brighter range (higher reflectance range).

It is assumed that exinite is negligible, since a ratio of exinite present is very low compared to vitrinite and inert.

In addition, in the present embodiment, the microstructural component group (vitrinite or inert) of the coal particle 9 included in the surface image 8 is identified based on the magnitude relationship in reflectance between vitrinite and inert. Specifically, the identification is carried out as follows.

The identification unit 3 of the coal analyzer 1 roughly determines the threshold of a vitrinite reflectance range in the aggregate reflectance histogram of all the coal particles. Specifically, as illustrated in FIG. 4, a minimum threshold 13 and a maximum threshold 14 of the vitrinite reflectance range are determined (step S104).

As a method of determining the thresholds, it is preferable to determine portions where the reflectance histogram changes rapidly as the thresholds. When this method is used, for example, the thresholds can be determined based on the differential value or the curvature of the reflectance histogram.

Next, the identification unit 3 of the coal analyzer 1 fits a normal distribution to the reflectance histogram of an individual coal particle (refer to FIG. 5 to be described later) on the assumption that the reflectance distribution of vitrinite is a normal distribution (step S105). The vitrinite reflectance range of the individual coal particle is thus determined.

Although the aggregate reflectance histogram of all the coal particles (refer to FIG. 4) may be used to determine the vitrinite reflectance range, the vitrinite reflectance range varies for each individual coal particle in reality. For this reason, determining the vitrinite reflectance range for each individual coal particle can make the finally obtained total inert amount more accurate.

FIG. 5 is a reflectance histogram of one coal particle.

In FIG. 5, a normal distribution 16 is fitted to a reflectance histogram 15 of one coal particle. The identification unit 3 of the coal analyzer 1 calculates an average u and a standard deviation σ, which are parameters of the normal distribution 16 (step S106).

FIG. 5 illustrates a minimum value 17 and a maximum value 18 of the vitrinite reflectance. The minimum value 17 of the vitrinite reflectance is obtained from the formula "u - 3σ". The maximum value 18 of the vitrinite reflectance is obtained from the formula "u + 3σ".

FIG. 5 also illustrates the thresholds (minimum threshold 13 and maximum threshold 14) of the previously determined vitrinite reflectance range.

In a case where minimum threshold 13 < u < maximum threshold 14 is satisfied (Y in step S107), the identification unit 3 of the coal analyzer 1 determines the vitrinite reflectance and the inert reflectance of the coal particle based on the following formulae (step S108). $u - 3 σ < vitrinite reflectance \leq u + 3 σ$ $u + 3 σ < inert reflectance$

Then, the microstructural component group (vitrinite or inert) is identified based on the reflectance for each pixel (step S110).

On the other hand, in a case where minimum threshold 13 < u < maximum threshold 14 is not satisfied (N in step S107), the identification unit 3 of the coal analyzer 1 determines the vitrinite reflectance and the inert reflectance of the coal particle based on the following formulae (step S109). 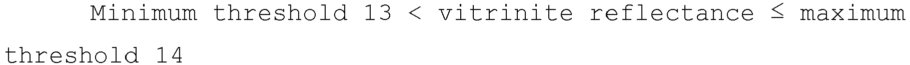 Minimum threshold 13 < vitrinite reflectance ≤ maximum threshold 14 $Maximum threshold 14 < inert reflectance$

Then, the microstructural component group (vitrinite or inert) is identified based on the reflectance for each pixel (step S110).

The identification unit 3 of the coal analyzer 1 identifies a microstructural component group for each pixel of all the coal particles 9 included in the surface image 8 of the coal sample 7.

Reflectance outside the range of the above formulae is not considered.

### <<Calculation unit>>

Next, the calculation unit 4 of the coal analyzer 1 calculates the average reflectance Ro (unit:%) of vitrinite based on the following formula (step S111). Hereinafter, the "average reflectance Ro of vitrinite" is also simply referred to as "Ro". Ro represents the degree of maturation of coal. 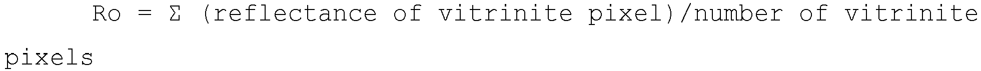 Ro = Σ (reflectance of vitrinite pixel)/number of vitrinite pixels

The "vitrinite pixel" means a pixel identified as vitrinite. In addition, an "inert pixel" means a pixel identified as inert.

Furthermore, the calculation unit 4 of the coal analyzer 1 calculates the total inert amount (unit:%) based on the following formula (step S112). 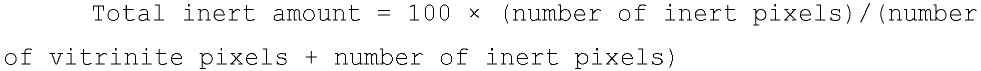 Total inert amount = 100 × (number of inert pixels)/(number of vitrinite pixels + number of inert pixels)

Also, when the ratio of the microstructural component group other than inertinite (for example, vitrinite) is calculated, it is obtained in the same manner as described above.

Here, a conventional method for obtaining the total inert amount will be described.

In the conventional method, in accordance with JIS M 8816-1992, the microstructural components of coal in the field of view are identified and counted based on a criterion such as a pattern while the stage of a microscope on which the coal sample is placed is manually moved at regular intervals.

Then, the total inert amount (unit:%) is obtained based on the following formula. 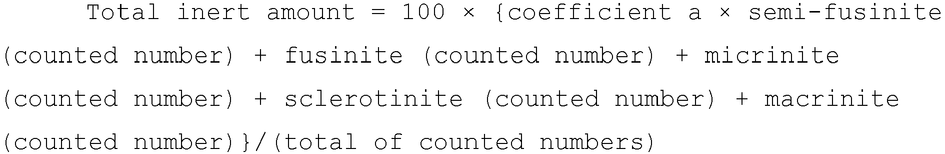 Total inert amount = 100 × {coefficient a × semi-fusinite (counted number) + fusinite (counted number) + micrinite (counted number) + sclerotinite (counted number) + macrinite (counted number)}/(total of counted numbers)

The coefficient a is 1 or 2/3 in consideration of the active component of semi-fusinite. The calculation herein uses 1 as the coefficient a, but the present invention is not limited thereto.

In the conventional method, Ro is also manually obtained in the same manner as in the case of the total inert amount.

That is, in accordance with JIS M 8816-1992, the average reflectance of vitrinite is obtained by manually moving the stage of the microscope and identifying the vitrinite in the field of view.

In such a conventional method, microstructural components of coal need to be individually identified, thereby making the method very complicated. In addition, since the identification is performed based on a criterion such as a pattern, the identification result is largely dependent on a person, and such identification is also time-consuming.

Although a "computer" is used in the method described in Patent Literature 1, microstructural components such as semi-fusinite and fusinite belonging to inertinite are individually identified based on a criterion such as "fluctuation range of reflectance", which also makes the method complicated.

On the other hand, according to the coal analyzer 1 (coal analysis method) of the present embodiment, it is possible to easily obtain the total inert amount, which is the ratio of one type of microstructural components, without individually identifying the microstructural components of coal. At this time, since a criterion such as a pattern is not used, the analyzer can depend less on a person and also reduce the time.

### <<Correction unit>>

Next, the correction unit 5 of the coal analyzer 1 corrects the total inert amount calculated by the calculation unit 4 (step S113).

Here, the reason for performing the correction will be described.

Hereinafter, for the sake of convenience, Ro and the total inert amount obtained using the coal analyzer 1 (coal analysis method) of the present embodiment may be referred to as "Ro (automatic)" and the "total inert amount (automatic)", respectively.

In addition, Ro and the total inert amount obtained by the conventional method may be referred to as "Ro (manual)" and the "total inert amount (manual)", respectively.

The present inventors compared Ro (automatic) and the total inert amount (automatic) with Ro (manual) and the total inert amount (manual).

Ro (manual) and the total inert amount (manual) were obtained from observation results of arbitrary 100 fields of view and 250 fields of view, respectively.

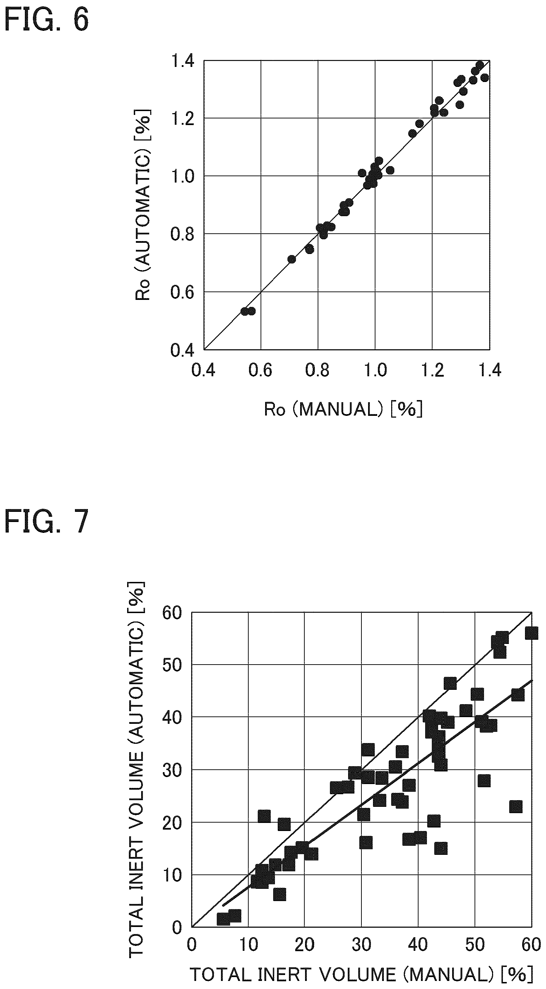

FIG. 6 is a relationship diagram between Ro (manual) and Ro (automatic). The coefficient of determination R² = 0.95 and the error standard deviation RMSE = 0.03% were found, and thus Ro (automatic) was equivalent to Ro (manual).

FIG. 7 is a relationship diagram between the total inert amount (manual) and the total inert amount (automatic). The coefficient of determination R² = 0.2 and the error standard deviation RMSE = 10% were found, and thus the total inert amount (automatic) tended to be underestimated in comparison with the total inert amount (manual).

In general, it is known that semi-fusinite belonging to inertinite has an intermediate property between vitrinite and inertinite.

Since the reflectance of semi-fusinite also indicates an intermediate value between those of vitrinite and inertinite, it is difficult to strictly classify semi-fusinite only by the magnitude of the reflectance value.

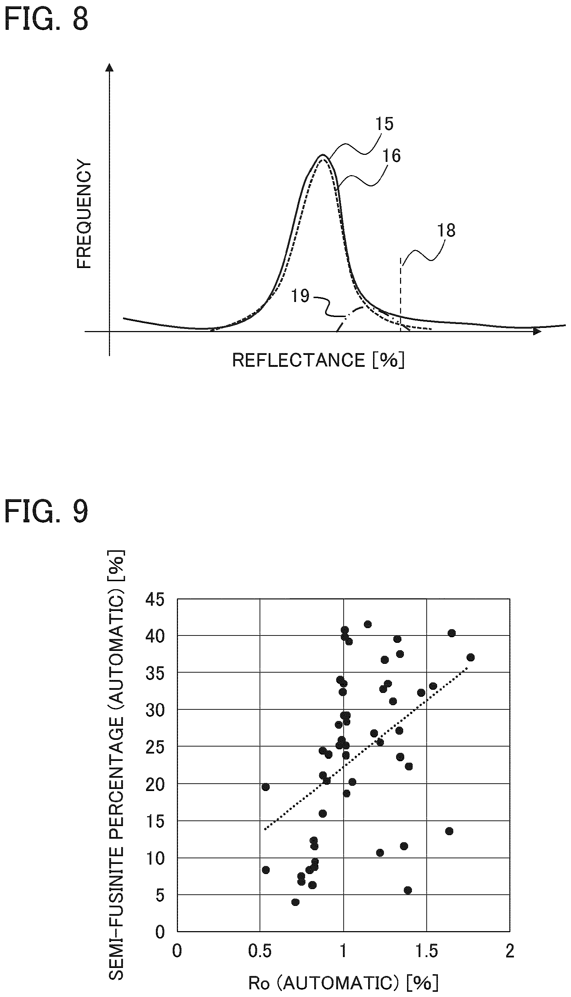

FIG. 8 is a reflectance histogram of one coal particle, which histogram illustrates a semi-fusinite reflectance. In FIG. 8, the normal distribution 16 is fitted to the reflectance histogram 15 of one coal particle, and the maximum value 18 of the vitrinite reflectance is also indicated.

Furthermore, FIG. 8 illustrates a semi-fusinite reflectance 19 (a histogram of the semi-fusinite reflectance).

As illustrated in FIG. 8, the vitrinite reflectance range includes many semi-fusinite reflectances. In this case, since semi-fusinite belonging to inertinite is erroneously recognized as vitrinite, the total inert amount (automatic) is underestimated in comparison with the total inert amount (manual), as illustrated in FIG. 7. The present inventors considered as described above.

Accordingly, the present inventors obtained the ratio (unit:%) of semi-fusinite by the conventional method as in the case of the total inert amount. Hereinafter, this may be referred to as a "semi-fusinite ratio (manual)".

FIG. 9 is a relationship diagram between Ro (automatic) and the semi-fusinite ratio (manual).

There is a correlation of the coefficient of determination R² = 0.3 between Ro (automatic) and the semi-fusinite ratio (manual), and as illustrated in FIG. 9, the semi-fusinite ratio (manual) increases as Ro (automatic) rises.

Based on FIG. 9 as described above, the present inventor considered that the influence of erroneously recognizing semi-fusinite as vitrinite when obtaining the total inert amount (automatic) can be corrected using Ro (automatic).

The present inventors performed multiple regression analysis on the total inert amount (manual) based on the following formula using two explanatory variables, Ro (automatic) and total inert amount (automatic). 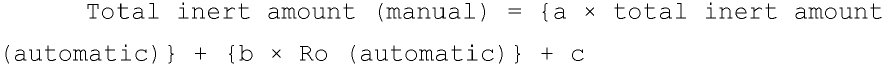 Total inert amount (manual) = {a × total inert amount (automatic)} + {b × Ro (automatic)} + c

The multiple regression coefficients were a = 0.92, b = 24.1, and c = -15.9.

The present inventors corrected the total inert amount (automatic) using the multiple regression coefficients obtained by the multiple regression analysis.

Specifically, a corrected total inert amount (automatic) was obtained based on the following formula. 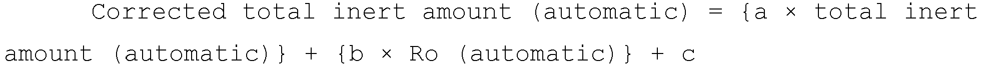 Corrected total inert amount (automatic) = {a × total inert amount (automatic)} + {b × Ro (automatic)} + c

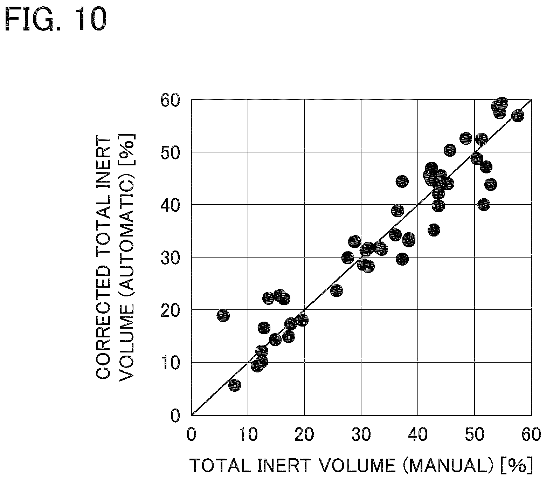

FIG. 10 is a relationship diagram between the total inert volume (manual) and the corrected total inert amount (automatic). The coefficient of determination R² = 0.9 and the error standard deviation RMSE = 4% were found, and thus the corrected total inert amount (automatic) was equivalent to the total inert amount (manual).

If the total inert amount (manual) is more accurate than the total inert amount (automatic), it can be said that the corrected total inert amount (automatic) obtained by the correction using the multiple regression coefficient is also accurate.

Therefore, the correction unit 5 of the coal analyzer 1 determines and retains the multiple regression coefficient in advance.

Then, the total inert amount is corrected using the multiple regression coefficient as well as Ro (automatic) and the total inert amount (automatic) calculated by the calculation unit 4 (step S113).

Through such correction, the finally obtained total inert amount can be made more accurate.

Note that the explanatory variables in the multiple regression analysis are not limited to the two, i.e., Ro (automatic) and the total inert amount (automatic). Other information that can be acquired from the surface image 8 may be further added as an explanatory variable.

### [Preparation of mixed coal and coke production]

Coke is produced from coal with various brands blended according to its product indexes.

That is, a plurality of types of coals are blended to prepare mixed coal, and the prepared mixed coal is calcined in a coke oven or the like to obtain coke.

At this time, it is preferable to identify the total inert amount of at least part of the coal to be blended using the coal analysis method of the present embodiment described above.

As described above, according to the coal analysis method of the present embodiment, the total inert amount can be easily obtained and the time can be shortened, and thus the preparation of mixed coal and coke production can also be simplified and expedited as a whole. In addition, the obtained total inert amount can be immediately reflected in coal blending calculation or the like.

### [REFERENCE SIGNS LIST]

- 1:: coal analyzer
- 2:: image acquisition unit
- 3:: identification unit
- 4:: calculation unit
- 5:: correction unit
- 6:: microscope
- 7:: coal sample
- 8:: surface image
- 9 (9a, 9b, 9c):: coal particle
- 10:: resin
- 11:: vitrinite
- 12:: inert
- 13:: minimum threshold in vitrinite reflectance range
- 14:: maximum threshold in vitrinite reflectance range
- 15:: reflectance histogram of one coal particle
- 16:: normal distribution
- 17:: minimum value of vitrinite reflectance
- 18:: maximum value of vitrinite reflectance
- 19:: semi-fusinite reflectance

## Claims

1. A coal analyzer (1) comprising:
an image acquisition unit (2) that acquires a surface image of a coal sample;
an identification unit (3) that identifies a microstructural component group included in the surface image for each pixel of the image, based on reflectance;
a calculation unit (4) that calculates a ratio of at least one of the microstructural component groups; and
a correction unit (5),
wherein the calculation unit (4) calculates a total inert amount that is a ratio of inertinite among the microstructural component groups, wherein inertinite is one of the microstructural components,
wherein the calculation unit (4) calculates an average reflectance Ro of vitrinite, which is one of the microstructural component groups from pixels identified as vitrinite, wherein the total inert amount is calculated from the number of pixels identified as inertinite and the number of pixels identified as vitrinite, and
wherein the correction unit (5) determines a corrected total inert amount from the total inert amount and the average reflectance Ro of vitrinite.

2. The coal analyzer according to claim 1, wherein
the correction unit (5) corrects the total inert amount using a multiple regression coefficient that is determined in advance, with the total inert amount and the average reflectance Ro of vitrinite as variables.

3. A coal analysis method comprising:
acquiring a surface image of a coal sample;
identifying a microstructural component group included in the surface image for each pixel of the image based on reflectance;
calculating a ratio of at least one of the microstructural component groups;
calculating a total inert amount that is a ratio of inertinite among the microstructural component groups, wherein inertinite is one of the microstructural components,
calculating an average reflectance Ro of vitrinite, which is one of the microstructural component groups from pixels identified as vitrinite, wherein the total inert amount is calculated from the number of pixels identified as inertinite and the number of pixels identified as vitrinite, and
determining a corrected total inert amount from the total inert amount and the average reflectance Ro of vitrinite.

4. The coal analysis method according to claim 3, further comprising correcting the total inert amount using a multiple regression coefficient that is determined in advance, with the total inert amount and the average reflectance Ro of vitrinite as variables.

5. A mixed coal preparation method comprising:
blending multiple types of coals to prepare mixed coal; and
identifying a total inert amount of at least part of the coal using the method according to any one of claims 3 to 4.

6. A coke production method comprising using the mixed coal prepared by the method according to claim 5 to obtain coke.

## Patentansprüche

1. Kohleanalysegerät (1), umfassend:
eine Bilderfassungseinheit (2), die ein Oberflächenbild einer Kohleprobe erfasst;
eine Identifizierungseinheit (3), die eine in dem Oberflächenbild enthaltene Mikrostrukturkomponentengruppe für jedes Pixel des Bildes auf der Grundlage von Reflexionsvermögen identifiziert;
eine Berechnungseinheit (4), die einen Anteil von mindestens einer der Mikrostrukturkomponentengruppen berechnet; und
eine Korrektureinheit (5),
wobei die Berechnungseinheit (4) eine Gesamtinertmenge berechnet, die ein Anteil von Inertinit unter den Mikrostrukturkomponentengruppen ist, wobei Inertinit eine der Mikrostrukturkomponenten ist,
wobei die Berechnungseinheit (4) ein mittleres Reflexionsvermögen Ro von Vitrinit, der eine der Mikrostrukturkomponentengruppen ist, aus als Vitrinit identifizierten Pixeln berechnet, wobei die Gesamtinertmenge aus der Anzahl der als Inertinit identifizierten Pixel und der Anzahl der als Vitrinit identifizierten Pixel berechnet wird, und
wobei die Korrektureinheit (5) eine korrigierte Gesamtinertmenge aus der Gesamtinertmenge und dem mittleren Reflexionsvermögen Ro von Vitrinit bestimmt.

2. Kohleanalysegerät nach Anspruch 1,
wobei die Korrektureinheit (5) die Gesamtinertmenge unter Verwendung eines im Voraus bestimmten multiplen Regressionskoeffizienten korrigiert, mit der Gesamtinertmenge und dem mittleren Reflexionsvermögen Ro von Vitrinit als Variablen.

3. Kohleanalyseverfahren, umfassend:
Erfassen eines Oberflächenbildes einer Kohleprobe;
Identifizieren einer in dem Oberflächenbild enthaltenen Mikrostrukturkomponentengruppe für jedes Pixel des Bildes auf der Grundlage von Reflexionsvermögen;
Berechnen eines Anteils von mindestens einer der Mikrostrukturkomponentengruppen;
Berechnen einer Gesamtinertmenge, die ein Anteil von Inertinit unter den Mikrostrukturkomponentengruppen ist, wobei Inertinit eine der Mikrostrukturkomponenten ist,
Berechnen eines mittleren Reflexionsvermögens Ro von Vitrinit, der eine der Mikrostrukturkomponentengruppen ist, aus als Vitrinit identifizierten Pixeln, wobei die Gesamtinertmenge aus der Anzahl der als Inertinit identifizierten Pixel und der Anzahl der als Vitrinit identifizierten Pixel berechnet wird, und
Bestimmen einer korrigierten Gesamtinertmenge aus der Gesamtinertmenge und dem mittleren Reflexionsvermögen Ro von Vitrinit.

4. Kohleanalyseverfahren nach Anspruch 3, weiter umfassend das Korrigieren der Gesamtinertmenge unter Verwendung eines im Voraus bestimmten multiplen Regressionskoeffizienten, mit der Gesamtinertmenge und dem mittleren Reflexionsvermögen Ro von Vitrinit als Variablen.

5. Mischkohleherstellungsverfahren, umfassend:
Mischen mehrerer Arten von Kohlen, um Mischkohle herzustellen; und
Identifizieren einer Gesamtinertmenge von mindestens einem Teil der Kohle unter Verwendung des Verfahrens nach einem der Ansprüche 3 bis 4.

6. Koksherstellungsverfahren, umfassend das Verwenden der durch das Verfahren nach Anspruch 5 hergestellten Mischkohle, um Koks zu erhalten.

## Revendications

1. Analyseur de charbon (1) comprenant :
une unité (2) d'acquisition d'image qui acquiert une image de surface d'un échantillon de charbon ;
une unité d'identification (3) qui identifie un groupe de composants microstructuraux inclus dans l'image de surface pour chaque pixel de l'image, sur la base de la réflectance ;
une unité de calcul (4) qui calcule une proportion d'au moins un des groupes de composants microstructuraux ; et
une unité de correction (5),
dans lequel l'unité de calcul (4) calcule une teneur totale en inertinite qui est une proportion d'inertinite parmi les groupes de composants microstructuraux, dans lequel l'inertinite est l'un des composants microstructuraux,
dans lequel l'unité de calcul (4) calcule une réflectance moyenne Ro de la vitrinite, laquelle est l'un des groupes de composants microstructuraux, à partir de pixels identifiés comme vitrinite, dans lequel la teneur totale en inertinite est calculée à partir du nombre de pixels identifiés comme inertinite et du nombre de pixels identifiés comme vitrinite, et
dans lequel l'unité de correction (5) détermine une teneur totale en inertinite corrigée à partir de la teneur totale en inertinite et de la réflectance moyenne Ro de la vitrinite.

2. Analyseur de charbon selon la revendication 1,
dans lequel l'unité de correction (5) corrige la teneur totale en inertinite en utilisant un coefficient de régression multiple qui est déterminé à l'avance, la teneur totale en inertinite et la réflectance moyenne Ro de la vitrinite étant utilisées comme variables.

3. Procédé d'analyse de charbon comprenant :
l'acquisition d'une image de surface d'un échantillon de charbon ;
l'identification d'un groupe de composants microstructuraux inclus dans l'image de surface pour chaque pixel de l'image sur la base de la réflectance ;
le calcul d'une proportion d'au moins un des groupes de composants microstructuraux ;
le calcul d'une teneur totale en inertinite qui est une proportion d'inertinite parmi les groupes de composants microstructuraux, dans lequel l'inertinite l'un des composants microstructuraux,
le calcul d'une réflectance moyenne Ro de la vitrinite, laquelle est l'un des groupes de composants microstructuraux, à partir de pixels identifiés comme vitrinite, la teneur totale en inertinite étant calculée à partir du nombre de pixels identifiés comme inertinite et du nombre de pixels identifiés comme vitrinite, et
la détermination d'une teneur totale en inertinite corrigée à partir de la teneur totale en inertinite et de la réflectance moyenne Ro de la vitrinite.

4. Procédé d'analyse de charbon selon la revendication 3, comprenant en outre l'étape consistant à corriger la teneur totale en inertinite en utilisant un coefficient de régression multiple qui est déterminé à l'avance, la teneur totale en inertinite et la réflectance moyenne Ro de la vitrinite étant prises comme variables.

5. Procédé de préparation de charbon mélangé comprenant :
le mélange de plusieurs types de charbons pour préparer du charbon mélangé ;
et
l'identification d'une teneur totale en inertinite d'au moins une partie du charbon en utilisant le procédé selon l'une quelconque des revendications 3 à 4.

6. Procédé de production de coke comprenant l'étape consistant à utiliser le charbon mélangé préparé par le procédé selon la revendication 5 pour obtenir du coke.
